# EUROPEAN PATENT SPECIFICATION

(11) **EP 0 497 389 B1**
(45) Date of publication and mention of the grant of the patent: **02.11.1995**
(21) Application number: 92107161.9
(22) Date of filing: 14.07.1987
(51) Int. Cl.: A61B 17/10, A61B 17/064, A61B 17/068

(54) **Staple closing mechanism with split actuator**
Klammerschliessvorrichtung mit gespaltenem Stössel
Farneur portagé pour agrofe

(30) Priority: 14.07.1986 US 885221
(43) Date of publication of application: 05.08.1992
(62) Divisional of application: 87306220.2
(73) Proprietor: MINNESOTA MINING AND MANUFACTURING COMPANY, St. Paul, Minnesota 55133-3427 (US)
(72) Inventor: Froehlich, Harold E., c/o Minnesota Mining and, St. Paul, Minnesota 55144-1000 (US); Foslien, Floyd L., c/o Minnesota Mining and, St. Paul, Minnesota 55144-1000 (US)
(74) Representative: Baillie, Iain Cameron

(56) References cited:
- EP-A- 0 061 867
- EP-A- 0 085 930
- EP-A- 0 094 752
- EP-A- 0 229 453
- WO-A-79/00970
- WO-A-82/02486
- US-A- 4 403 693
- US-A- 4 470 532

## Description

### Technical Field

The present invention relates to the shapes of rams and anvils used to close open wire staples for suturing living tissue.

### Background Art

The art is replete with shapes of open wire staples for suturing living tissues, and rams and anvils of various shapes used to close such staples. U.S. Patent Nos. 3,873,016; 4,202,480; 4,256,251; 4,321,002; 4,477,007; 4,477,008; and 4,519,532 provide illustrative examples.

Such prior art open wire staples have included a staple having a straight central portion with straight pointed end portions projecting at right angles thereto (such as is illustrated in Figure 4 of U.S. Patent No. 4,202,480, which staple may be bent at two spaced locations along its central portion to form a generally rectangular closed staple (see Figure 6 of U.S. Patent No. 4,202,480) and is called a "box" staple herein. The side of the closed box staple opposite its end portions can subsequently be bent into a U-shape to retract the staple from the skin and underlying tissue as is illustrated in U.S. Patent 4,026,520. A further box staple is disclosed in EP-A-0229453 comprising a generally U-shaped central portion connected to an intermediate portion with an angle of 100-110° and the outer end of the intermediate portion is joined by an acute angle of 70-75° to outer legs which are parallel to one another and which terminate in points. The European application falls under Article 54(3) EPC. Such box staples have been widely used and have been well accepted by the medical community. This is at least partially so because of the currently preferred method for stapling skin. It is known that the inner surfaces of skin edge portions separated by an injury or an incision must make contact for proper healing to occur, and that if skin edge portions are inverted during stapling (i.e., positioned with their outer surfaces face to face) poor healing with the probability of scarring will result. Thus skin edge portions to be stapled together typically are everted (i.e., tented upwardly) with the inner surface of the skin edge portions pressed together, and the staple is applied over the everted skin edge portions to hold them together. When the box staple is so used, the parts of the skin edge portions pierced by the staple initially are positioned along its aligned end portions on the side of the closed box staple adjacent the skin portions being stapled. With time, tensions in the skin transverse to the opening being stapled closed will cause the parts of the skin portion pierced by the staple to move from around the adjacent end portions (which then become buried in tissue beneath the joined skin portions) to positions around the opposite side portions of the closed staple at which the skin edge portions lose some of their original eversion and are more in alignment. With the box staple, however, this movement of the skin does not occur immediately due to the restriction caused by the right angle corners between the end portions and side portions of the staple around which corners those parts of the skin portions must pass. Thus, the skin portions have a tendency to remain everted along the entire length of the opening being closed at least for the period of time required to complete stapling together of the skin edge portions, and a person who has completed the stapling can visually assure himself after the stapling of the skin edge portions is complete that the skin edge portions are still in a position which will afford proper healing. The box type staple, however, suffers from the disadvantage of requiring the staple to be bent sharply in two locations to close it in a manner which has required the use of metal rams and anvils and the application of substantial closing force; and causes some damage to the skin, particularly when the parts of the skin edge portions pierced by the staple move around the right angle bends in the staple as described above.

Another prior art staple for suturing living tissue described in U.S. Patent No. 4,321,002 comprises a generally U-shaped central portion having at least one arcuate part and curved outer portions terminating in sharp points. That staple can be closed by bending generally straight the arcuate part or parts so that the curved outer portions will smoothly enter and gather living tissue such as skin portions. During such closure the sharp points on the outer portions move to adjacent generally aligned positions to provide a generally D-shaped staple. As with the previously described box staple, the central portion of the closed D-shaped staple can subsequently be bent to retract the outer portions of the staple from the tissue.

The force needed to close such a D-shaped staple having only one arcuate part is substantially reduced for a similarly sized staple from the force required to close the box staple described above, so that the D-shaped staple can be closed with a ram and anvil made of polymeric material such as is described in U.S. Patents No's. 4,477,008 and 4,519,532 (incorporated herein by reference). Also the curved outer portions can cause a reduced amount of damage to skin and tissue during use compared to the box staple. Such D-shaped staples, however, have not been received by the medical community with the enthusiasm one might expect presumably in part because the closed D-shaped staple will not hold joined skin portions in an everted position as long as a closed box staple. The stapled skin portions easily and quickly move along the arcuate portions of the D-shaped staple to a generally aligned position (which aligned position provides as good or better healing than the everted position) typically before the person stapling together the skin edge portions has completed the required stapling, so that that person can not observe all of the stapled skin edge portions in an everted position and thus be sure that some of the skin portions will not subsequently become inverted.

Also, even though the closing force of the "D" shaped staple having only one arcuate part is sufficiently low that rams and anvils used to close it can be made of polymeric material, significant wear can occur on such polymeric rams and anvils when they are used to close a large number of staples; and it has been found that staples can slip sideways along the surfaces of the polymeric rams and anvils contacting the staple (a phenomenon not observed between metal anvils, rams and staples) which can result in improperly shaped closed staples.

Another general problem that has existed in the design of rams and anvils for staplers has been to deal with the tendency for the central portion of the open staple to bend away from the anvil toward the ram as a result of forces by which the staple is bent to its closed position. One suggested approach for use with box staples has been to arch the center portion of the open staple toward the anvil so that it bends to a generally straight position as the staple is bent closed as is illustrated in U.S. Patent No. 4,256,251 (see Figures 12 and 19). Another approach being used is to shape the central portion of the ram so that it will straighten the bowed central portion of the staple by bending it against the anvil after the staple is essentially closed as is done in the "Precise"^{∼} stapler sold for several years by Minnesota Mining and Manufacturing Company, St. Paul, Minnesota. Yet another approach has been to provide a support to prevent such bending on the side of the central portion of the staple opposite the ram as is described in U.S. Patent No. 4,127,227.

However prevented, it is preferable that the central portion of the staple is not bowed significantly upwardly after the staple is closed, since if such a bow is present, pressure from the jaws of a staple remover of the type described in U.S. Patent No. 4,026,520 will cause crank-like forces tending to rotate the staple around its central portion as it is bent open, which rotation of the staple can cause the staple to be bent out of its plane which may damage the tissue from which the staple is being removed.

It is an object of the present invention to provide a ram and anvil, which can be made of polymeric materials, by which the staple as claimed in co-pending application EP-A-0253629 or the D-shaped staple described above can be bent closed without slipping sideways on the anvil and with substantially less initial closing force than is needed with prior art ram and anvil combinations, and which applies forces in a manner that reduces the tendency for the central portion of the staple to bend away from the anvil as it is closed.

According to one aspect of this invention there is provided a stapler adapted to close an open wire staple for suturing living tissue of the type comprising a generally U-shaped central portion having opposite ends and at least one arcuate part; and outer portions terminating in sharp points, the parts of each outer portion opposite said sharp point being disposed at about a right angle to and being connected at a first corner to one end of said central portion, said staple being closable by bending generally straight the arcuate part of said central portion adjacent each of said outer portions so that said outer portions can enter and gather living tissue, during which closure the sharp points on said outer portions move to adjacent positions generally aligned with each other to provide a closed staple, said stapler comprising:
an anvil having spaced support surfaces and a recess between said support surfaces adapted to receive a central part of the generally U-shaped central portion of the staple;
a ram comprising an end portion of resiliently flexible material having two separated parts each having an end surface adapted to engage the open staple, said end surfaces having spaced outer portions and inner portions recessed from said outer portions and extending generally transverse of said ram, said end surfaces being arcuate from said outer portions to said inner portions to form opposed arcuate surface portions adapted to engage along the outer portions of the open staple, the junctures between said arcuate surface portions and said inner surface portions forming sockets adapted to engage the first corners of the staple when its central portion is partially straightened and said inner surface portions being disposed to straighten the central portion across said anvil to complete closure of the staple; and
means for moving said ram toward said anvil to close the staple around said anvil with the central portion of the staple supported against said spaced support surfaces by sequentially pressing said arcuate end surface portions against the outer portions of the staple to resiliently spread said parts of said ram and apply forces both longitudinally and transversely of said ram to partially straighten the central portion of the staple to subsequently press the surfaces forming said sockets against the first corners of the staple to substantially straighten the central portion of the staple; and to then engage said inner surface portions of said ram with the central portion of the staple to straighten the central portion and complete closure of the staple.

According to a further feature of the invention there is provided a stapler comprising:
an open wire staple for suturing living tissue, said open staple comprising a generally U-shaped central portion having opposite ends and at least one arcuate part; and outer portions terminating in sharp points, parts of each outer portion opposite said sharp point being disposed at about a right angle to and being connected at a first corner to one end of said central portion, said staple being closable be bending generally straight the arcuate part of said central portion adjacent each of said outer portion so that said outer portions can enter and gather living tissue, during which closure the sharp points on said outer portions move to adjacent positions generally aligned with each other to provide a closed staple;
an anvil having spaced support surfaces and a recess between said support surfaces adapted to receive a central part of the generally U-shaped central portion of said staple;
a ram comprising an end portion of resiliently flexible material having two separated parts each having an end surface adapted to engage said open staple, said end surfaces having spaced outer portions and inner portions recessed from said outer portions and extending generally transverse of said ram, said end surfaces being arcuate from said outer portions to said inner portions to form opposed arcuate surface portions adapted to engage along the outer portions of said open staple, the junctures between said arcuate surface portions and said inner surface portions forming sockets adapted to engage said first corners of said staple when said central portion is partially straightened and said inner surface portions being disposed to straighten said central portion across said anvil to complete closure of said staple; and
means for moving said ram toward said anvil to close said staple around said anvil with the central portion of said staple supported against said spaced support surfaces by sequentially; pressing said arcuate end surface portions against said outer portions to resiliently spread said parts of said ram and apply forces both longitudinally and transversely of said ram to partially straighten said central portion; to subsequently press the surfaces forming said sockets against the first corners of said staple to substantially straighten said central portion; and to then engage said inner surface portions of said ram with said central portion to straighten said central portion and complete closure of said staple.

The anvil may be of polymeric material. The ram also may be of polymeric material.

The application of forces both longitudinally and transversely of the ram provides a resultant force which both closes the staple with less initial force applied to the ram than with a ram that does not have resiliently spreadable parts and in combination with the two point support of the anvil can straighten the central portion of a staple having one arcuate part with almost no tendency for the central part of it to bend away from the anvil toward the ram. The resiliency of the parts and the location of the arcuate end portions as they move along the staple may be selected so that the forces tending to straighten the U-shaped central portion of the staple are applied along lines generally parallel to the axes of the generally straight end parts of the U-shaped central portion and spaced from the axis of those end parts on the sides of those end parts opposite the center of the U-shaped portion a distance about equal the distance that would theoretically make such a force cause a long column to buckle, so that the forces instead tend to straighten the central part of the central portion. Tests have shown that straightening forces estimated using the column bending theoretical formulas in this way are very close to actual measured forces needed to straighten the central part in a staple.

Also, the application of forces both longitudinally and transversely by the separable parts of the ram and the spaced support surfaces on the anvil keep the staple essentially centered across the anvil and the ram so that a properly shaped closed staple results.

### Brief Description of the Drawings

The present invention will be further described with reference to the accompanying drawings wherein like reference numerals refer to like parts in the several views, and wherein:
Figure 1 is a plan view of the open wire staple for suturing living tissue, subject of co-pending application EP-A-0253629;
Figure 2 and 3 are plan views of the staple of Figure 1 closed in living tissue;
Figure 4 is a plan view of the staple of Figure 2 bent to an open position by a staple remover;
Figures 5 through 8 are fragmentary schematic views sequentially illustrating closing of the open wire staple of Figure 1 using a ram and anvil structure according to the present invention.

### Detailed Description

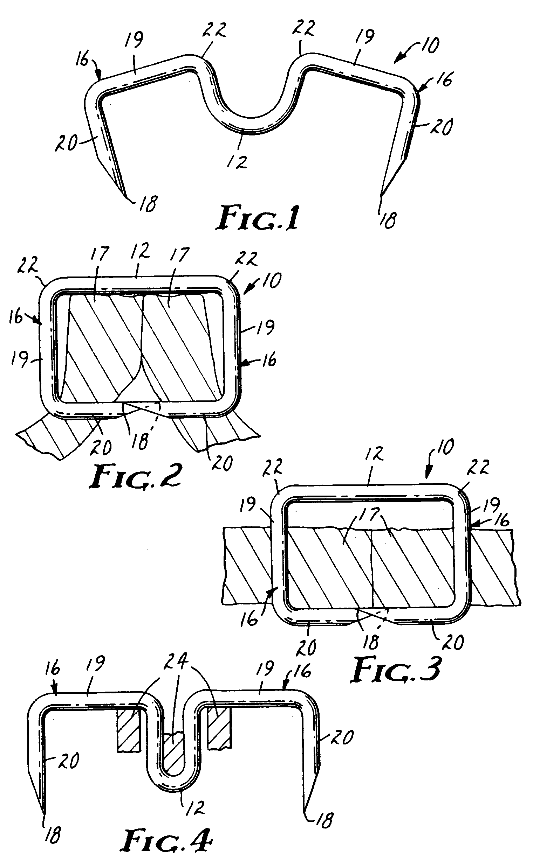

Referring now to Figure 1 of the drawing there is illustrated an unused open wire staple for suturing living tissue according to the co-pending application EP-A-0 253 629, generally designated by the reference numeral 10.

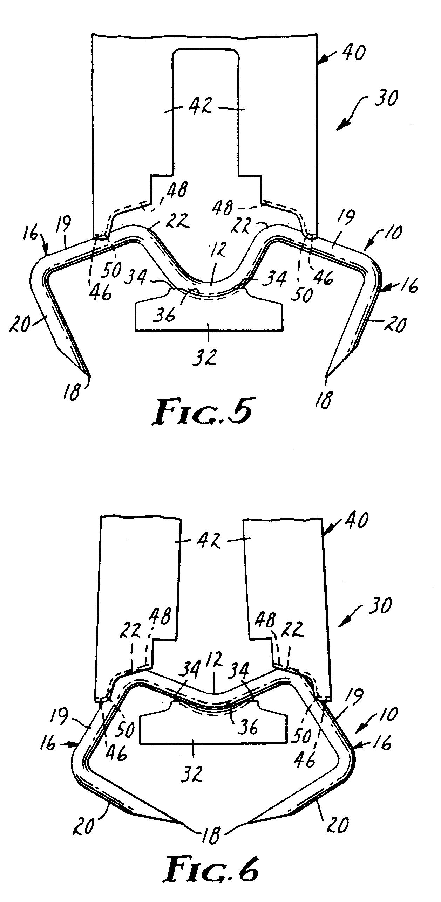

As is best seen in Figure 1, the open wire staple 10 comprises a generally U-shaped central portion 12 having one arcuate part; and outer portions 16 terminating in sharp points 18. The outer portions 16 each comprise a generally straight proximal part 19 and a generally straight distal part 20 with the parts 19 and 20 disposed at about a right angle to each other and with the sharp point 18 being on the end of each distal part 20 opposite the proximal part 19. The end of the proximal part 19 opposite the distal part 20 is disposed at about a right angle to and is connected at a first corner 22 to one end of the central portion 12. The staple is closable to the closed position shown in Figures 2 and 3 by bending generally straight the arcuate part of its central portion 12 so that the outer portions 16 can enter and gather living tissue such as adjacent skin edge portions 17. During such closure, the sharp points 18 on the distal parts 20 move to adjacent positions with the distal parts 20 generally aligned with each other to provide a generally rectangular or box shaped closed staple. Typically, the skin edge portions 17 are initially everted as shown in Figure 2 so that only the distal parts 20 pierce the skin edge portions 17. Subsequently, as described above in the Background Art portion of this specification, tension in the skin edge portions 17 causes them to move around the right angle corners between the distal parts 20 and proximal parts 19 to the generally aligned positions shown in Figure 3.

As shown in Figure 4, the central portion 12 of the closed staple can subsequently be bent to a U-shape by the jaws 24 of the type of staple remover described in U.S. Patent No. 4,026,520 to retract the outer portions 16 of the staple 10 from the skin portions 17.

The staple is preferably formed of a wire with a circular cross section (e.g. 0.57mm (0.0225 inch diameter)) and as can be seen in Figure 1, the central part of the central portion 12 of the unused open staple 10 is bent in a semicircular arc (e.g. 1.69mm (0.0666 inch)) radius to its outer surface). The outer parts of the central portion 12 diverge from parallel by about 30 degrees so that the proximal parts 19 of the staple 10 correspondingly are disposed to form an angle of about 150 degrees with each other. Alternatively the outer parts of the central portion 12 could be disposed between positions parallel with each other so that the proximal parts 19 are aligned, and portions diverging from parallel by about 60 degrees so that the proximal parts 19 form an angle of about 120 degrees with each other.

Referring now to Figures 5 through 8, there is schematically illustrated a stapler 30 for closing the staple 10, which stapler 30 may be in the form of the stapler illustrated in U.S. Patent No. 4,519,532

Generally, as illustrated, the stapler 30 comprises an anvil 32 having spaced support surfaces 34 and a recess 36 between the support surfaces 34 adapted to receive a central part of the generally U-shaped central portion 12 of the staple 10. Also, the stapler 30 comprises a ram 40 comprising an end portion of resiliently flexible material having two spaced and separated parts 42 each having a concave end surface adapted to engage the open staple 10. The end surfaces have spaced outer portions 46 and inner portions 48 recessed from the outer portions and extending generally transverse of the ram 40. The end surfaces are arcuate from their outer portions 46 to their inner portions 48 to form opposed arcuate surface portions 50 adapted to engage along the outer portions 16 of the open staple. The junctures between the surface portions 50 and the inner surface portions 48 form sockets adapted to engage the first corners 22 of the staple 10 when the central portion 12 is partially straightened and the inner surface portions 48 are disposed to straighten the central portion 12 across the anvil 32 when the staple 10 to complete closure of the staple 10.

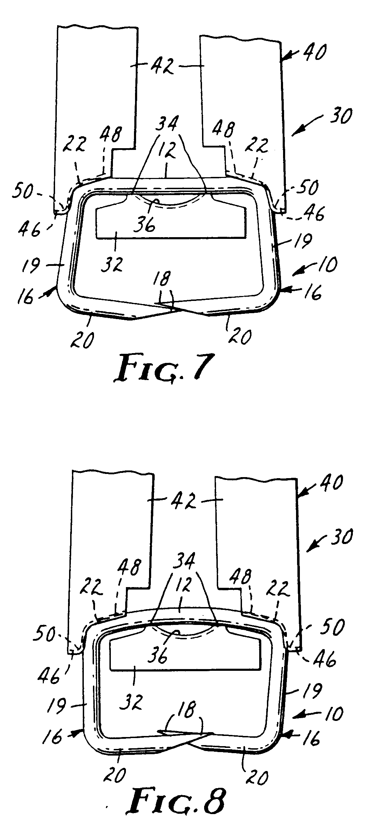

Also included in the stapler are means (not shown) for moving the ram 40 toward the anvil 32 to close the staple 10 around the anvil 32 with the central portion 12 of the staple 10 supported against the spaced support surfaces 34, which means may be in the form of the manually operable toggle joint linkage type drive described in U.S. Patent No. 4,519,532. Such movement of the ram 40 by such means will sequentially (1) press the radiused end surface portions 50 of the ram 40 against the proximal parts 19 of the outer portions 16 of the staple 10 (Figure 5) to resiliently spread the parts 42 of the ram 40 and apply forces both longitudinally and transversely of the ram 40 to generally straighten the central portion 12 of the staple (Figure 6); (2) to subsequently press the surfaces 50 and 48 forming the sockets against the first corners 22 of the staple 10 to generally straighten the central portion 12 (Figure 7); and (3) to then engage the inner surface portions 48 of the ram 40 with the central portion 12 of the staple to straighten the central portion 12 across the anvil 32 and complete closure of the staple 10.

The inner surface portions 48 of the ram are each disposed at a slight angle with respect to a line at a right angle to the longitudinal centerline of the ram 40 (e.g., 15 degrees) and are relieved opposite the support surfaces 34 which causes them to bend the central portion 12 of the staple into a slight arch around the support surfaces 34 of the anvil 32 (Figure 8). The elasticity in the staple wire will cause this arch to straighten when the pressure between the ram 40 and anvil 32 is removed, however, to provide a straight central portion 12 for the closed staple 10 as is shown in Figures 2 and 3.

Preferably the resiliency and geometry of the ram 40 are selected so that spreading of the parts 42 of the ram 40 during movement of the ram 40 between the positions shown in Figures 5 and 6 causes a resultant force to be applied to the proximal parts 19 of the staple 10 which is generally parallel to the axes of the generally straight end parts of the U-shaped central portion 12 and is spaced from the axis of those and parts on the sides of those end parts opposite the center of the U-shaped portion 12 a distance about equal the distance that would theoretically make such a force cause a long column the diameter of the staple 10 to buckle, so that the forces instead tend to straighten the central part of the central portion 12. While it is almost impossible to maintain precise force alignment of this type, it has been found that the closer such alignment can be maintained the less force is required to initially bend the staple 10 toward a closed position, which is particularly advantageous for a ram 40 driven by a toggle joint linkage type drive with which it is more difficult to develop force at the ram 40 during initial movement of the ram 40 than later in the movement of the ram 40.

The present invention has now been described with reference to one embodiment thereof. It will be apparent to those skilled in the art that many changes can be made in the embodiment described without departing from the scope of the present invention. For example, the generally U-shaped central portion of the staple 10 can alternately be shaped like the central portion 60 of the staple 13 described in U.S. Patent No. 4,321,002 to have two spaced arcuate parts 61. This provides a staple that can provide more gather to pull together skin portions it engages, however, the staple cannot be closed by the type of ram 40 and anvil 32 illustrated above without some bending of the center of its central portions away from the anvil 32 toward the ram 40.

## Claims

1. A stapler adapted to close an open wire staple (10) for suturing living tissue of the type comprising a generally U-shaped central portion (12) having opposite ends and at least one arcuate part; and outer portions (16) terminating in sharp points (18), the parts (19) of each outer portion (16) opposite said sharp point (18) being disposed at about a right angle to and being connected at a first corner (22) to one end of said central portion (12), said staple being closable by bending generally straight the arcuate part of said central portion (12) adjacent each of said outer portions (16) so that said outer portions (16) can enter and gather living tissue, during which closure the sharp points (18) on said outer portions (16) move to adjacent positions generally aligned with each other to provide a closed staple (10), said stapler comprising:
an anvil (32) having spaced support surfaces (34) and a recess (36) between said support surfaces (34) adapted to receive a central part of the generally U-shaped central portion (12) of the staple (10);
a ram (40) comprising an end portion of resiliently flexible material having two separated parts (42) each having an end surface adapted to engage the open staple (10), said end surfaces having spaced outer portions (46) and inner portions (48) recessed from said outer portions (46) and extending generally transverse of said ram (40), said end surfaces being arcuate from said outer portions (46) to said inner portions (48) to form opposed arcuate surface portions (50) adapted to engage along the outer portions (16) of the open staple (10), the junctures between said arcuate surface portions (50) and said inner surface portions (48) forming sockets adapted to engage the first corners (22) of the staple (10) when its central portion (12) is partially straightened and said inner surface portions (48) being disposed to straighten the central portion (12) across said anvil (32) to complete closure of the staple (10); and
means for moving said ram (40) toward said anvil (32) to close the staple (10) around said anvil (32) with the central portion (12) of the staple (10) supported against said spaced support surfaces (34) by sequentially;
(1) pressing said arcuate end surface portions (50) against the outer portions (16) of the staple (10) to resiliently spread said parts (42) of said ram (40) and apply forces both longitudinally and transversely of said ram (40) to partially straighten the central portion (12) of the staple (10);
(2) to subsequently press the surfaces (50, 48) forming said sockets against the first corners (22) of the staple (10) to substantially straighten the central portion (12) of the staple (10); and
(3) to then engage said inner surface portions (48) of said ram (40) with the central portion (12) of the staple (10) to straighten the central portion (12) and complete closure of the staple (10).

2. A stapler comprising:
an open wire staple (10) for suturing living tissue, said open staple (10) comprising a generally U-shaped central portion (12) having opposite ends and at least one arcuate part; and outer portions (16) terminating in sharp points (18), parts (19) of each outer portion (16) opposite said sharp point (18) being disposed at about a right angle to and being connected at a first corner (22) to one end of said central portion (12), said staple being closable be bending generally straight the arcuate part of said central portion (12) adjacent each of said outer portions (16) so that said outer portions (16) can enter and gather living tissue, during which closure the sharp points (18) on said outer portions (16) move to adjacent positions generally aligned with each other to provide a closed staple;
an anvil (32) having spaced support surfaces (34) and a recess (36) between said support surfaces (34) adapted to receive a central part of the generally U-shaped central portion (12) of said staple (10);
a ram (40) comprising an end portion of resiliently flexible material having two separated parts (42) each having an end surface adapted to engage said open staple (10), said end surfaces having spaced outer portions (46) and inner portions (48) recessed from said outer portions (46) and extending generally transverse of said ram (40), said end surfaces being arcuate from said outer portions (46) to said inner portions (48) to form opposed arcuate surface portions (50) adapted to engage along the outer portions (16) of said open staple (10), the junctures between said arcuate surface portions (50) and said inner surface portions (48) forming sockets adapted to engage said first corners (22) of said staple (10) when said central portion (20) is partially straightened and said inner surface portions (48) being disposed to straighten said central portion (12) across said anvil (32) to complete closure of said staple (10); and
means for moving said ram (40) toward said anvil (32) to close said staple (10) around said anvil (32) with the central portion (12) of said staple (10) supported against said spaced support surfaces (34) by sequentially;
(1) pressing said arcuate end surface portions (50) against said outer portions (16) to resiliently spread said parts (42) of said ram (40) and apply forces both longitudinally and transversely of said ram (40) to partially straighten said central portion (12);
(2) to subsequently press the surfaces (50, 48) forming said sockets against the first corners (22) of said staple (10) to substantially straighten said central portion (12); and
(3) to then engage said inner surface portions (48) of said ram (40) with said central portion (12) to straighten said central portion (12) and complete closure of said staple (10).

3. A stapler according to claim 1 or 2 wherein at least one of the anvil and the ram are made at least partly of polymeric material.

4. A stapler according to any preceding claim wherein the generally U-shaped central portion (12) of the staple (10) has generally straight end parts, and said arcuate end surfaces apply forces against the outer portions (16) of the staple (10) generally aligned with the end parts of the staple (10) and acting on the sides of those end parts opposite the center of said central portion (12).

## Patentansprüche

1. Heftmaschine, die eine offene Drahtklammer (10) zum Nähen von lebendem Gewebe schließen kann, umfassend einen im allgemeinen U-förmigen mittleren Abschnitt (12) mit einander entgegengesetzten Enden und mindestens einem gebogenen Teil; und äußere Abschnitte (16), die in scharfen Spitzen (18) enden, wobei die den scharfen Spitzen (18) entgegengesetzten Teile (19) jedes äußeren Abschnitts (16) etwa im rechten Winkel zu einem Ende des mittleren Abschnitts (12) angeordnet und an einer ersten Ecke (22) mit diesem verbunden sind, wobei die Klammer dadurch geschlossen werden kann, daß der gebogene Teil des mittleren Abschnitts (12) im Bereich jedes der äußeren Abschnitte (16) im allgemeinen geradegebogen wird, so daß die äußeren Abschnitte (16) in lebendes Gewebe eindringen und dieses zusammenhalten können, wobei sich die scharfen Spitzen (18) an den äußeren Abschnitten (16) bei diesem Schließvorgang in im allgemeinen miteinander fluchtende benachbarte Positionen bewegen, so daß man eine geschlossene Klammer (10) erhält, wobei die Heftmaschine folgendes umfaßt:
einen Amboß (32) mit voneinander beabstandeten Auflageflächen (34) und einer Vertiefung (36) zwischen den Auflageflächen (34), die einen mittleren Teil des im allgemeinen U-förmigen mittleren Abschnitts (12) der Klammer (10) aufnehmen kann;
einen Stößel (40) umfassend einen Endabschnitt aus elastisch flexiblem Material mit zwei getrennten Teilen (42), die jeweils eine Stirnfläche besitzen, die mit der offenen Klammer (10) in Eingriff treten kann, wobei die Stirnflächen voneinander beabstandete äußere Abschnitte (46) und innere Abschnitte (48) aufweisen, die gegenüber den äußeren Abschnitten (46) zurückgesetzt sind und sich im allgemeinen quer zu dem Stößel (40) erstrecken, wobei die Stirnflächen von den äußeren Abschnitten (46) zu den inneren Abschnitten (48) gebogen sind, um einander gegenüberliegende gebogene Oberflächenabschnitte (50) zu bilden, die entlang der äußeren Abschnitte (16) der offenen Klammer (10) in Eingriff treten können, wobei die Übergänge zwischen den gebogenen Oberflächenabschnitten (50) und den inneren Oberflächenabschnitten (48) Aufnahmen bilden, die mit den ersten Ecken (22) der Klammer (10) in Eingriff treten können, wenn ihr mittlerer Abschnitt (12) teilweise geradegebogen ist, und die inneren Oberflächenabschnitte (48) so angeordnet sind, daß der mittlere Abschnitt (12) quer über den Amboß (32) geradegebogen wird, um die Klammer (10) vollständig zu schließen; und
Mittel, um den Stößel (40) zu dem Amboß (32) zu bewegen, um die Klammer (10) um den Amboß (32) herum zu schließen, wobei der mittlere Abschnitt (12) der Klammer (10) gegen die voneinander beabstandeten Auflageflächen (34) gedrückt wird, indem nacheinander:
(1) die gebogenen Stirnflächenabschnitte (50) gegen die äußeren Abschnitte (16) der Klammer (10) gedrückt werden, um die Teile (42) des Stößels (40) elastisch auseinanderzudrücken und sowohl in Längsrichtung als auch in Querrichtung des Stößels (40) Kräfte auszuüben, durch die der mittlere Abschnitt (12) der Klammer (10) teilweise geradegebogen wird;
(2) anschließend die die Aufnahmen bildenden Flächen (50, 48) gegen die ersten Ecken (22) der Klammer (10) gedrückt werden, um den mittleren Abschnitt (12) der Klammer (10) im wesentlichen geradezubiegen; und
(3) dann die inneren Oberflächenabschnitte (48) des Stößels (40) mit dem mittleren Abschnitt (12) der Klammer (10) in Eingriff gebracht werden, um den mittleren Abschnitt (12) geradezubiegen und die Klammer (10) vollständig zu schließen.

2. Heftmaschine, umfassend:
eine offene Drahtklammer (10) zum Nähen von lebendem Gewebe, wobei die offene Klammer (10) einen im allgemeinen U-förmigen mittleren Abschnitt (12) mit einander entgegengesetzten Enden und mindestens einem gebogenen Teil umfaßt; und äußere Abschnitte (16), die in scharfen Spitzen (18) enden, wobei Teile (19) jedes der scharfen Spitze (18) entgegengesetzten äußeren Abschnitts (16) etwa im rechten Winkel zu einem Ende des mittleren Abschnitts (12) angeordnet und an einer ersten Ecke (22) mit diesem verbunden sind, wobei die Klammer geschlossen werden kann, indem der gebogene Teil des mittleren Abschnitts (12) im Bereich jedes der äußeren Abschnitte (16) im allgemeinen geradegebogen wird, so daß die äußeren Abschnitte (16) in lebendes Gewebe eindringen und dieses zusammenhalten können, wobei sich die scharfen Spitzen (18) an den äußeren Abschnitten (16) während dieses Schließvorgangs in im allgemeinen miteinander fluchtende benachbarte Positionen bewegen, so daß man eine geschlossene Klammer erhält;
einen Amboß (32) mit voneinander beabstandeten Auflageflächen (34) und einer Vertiefung (36) zwischen den Auflageflächen (34), die einen mittleren Teil des im allgemeinen U-förmigen mittleren Abschnitts (12) der Klammer (10) aufnehmen kann;
einen Stößel (40) umfassend einen Endabschnitt aus elastisch flexiblem Material mit zwei getrennten Teilen (42), die jeweils eine Stirnfläche besitzen, die mit der offenen Klammer (10) in Eingriff treten kann, wobei die Stirnflächen voneinander beabstandete äußere Abschnitte (46) und innere Abschnitte (48) aufweisen, die gegenüber den äußeren Abschnitten (46) zurückgesetzt sind und sich im allgemeinen quer zu dem Stößel (40) erstrecken, wobei die Stirnflächen von den äußeren Abschnitten (46) zu den inneren Abschnitten (48) gebogen sind, um einander gegenüberliegende gebogene Oberflächenabschnitte (50) zu bilden, die entlang der äußeren Abschnitte (16) der offenen Klammer (10) in Eingriff treten können, wobei die Übergänge zwischen den gebogenen Oberflächenabschnitten (50) und den inneren Oberflächenabschnitten (48) Aufnahmen bilden, die mit den ersten Ecken (22) der Klammer (10) in Eingriff treten können, wenn der mittlere Abschnitt (12) teilweise geradegebogen ist und die inneren Oberflächenabschnitte (48) so angeordnet sind, daß der mittlere Abschnitt (12) quer über den Amboß (32) geradegebogen wird, um die Klammer (10) vollständig zu schließen; und
Mittel, um den Stößel (40) zu dem Amboß (32) zu bewegen, um die Klammer (10) um den Amboß (32) herum zu schließen, wobei der mittlere Abschnitt (12) der Klammer (10) gegen die voneinander beabstandeten Auflageflächen (34) gedrückt wird, indem nacheinander:
(1) die gebogenen Stirnflächenabschnitte (50) gegen die äußeren Abschnitte (16) gedrückt werden, um die Teile (42) des Stößels (40) elastisch auseinanderzudrücken und sowohl in Längsrichtung als auch in Querrichtung des Stößels (40) Kräfte auszuüben, durch die der mittlere Abschnitt (12) teilweise geradegebogen wird;
(2) anschließend die die Aufnahmen bildenden Flächen (50, 48) gegen die ersten Ecken (22) der Klammer (10) gedrückt werden, um den mittleren Abschnitt (12) der Klammer (10) im wesentlichen geradezubiegen; und
(3) dann die inneren Oberflächenabschnitte (48) des Stößels (40) mit dem mittleren Abschnitt (12) in Eingriff gebracht werden, um den mittleren Abschnitt (12) geradezubiegen und die Klammer (10) vollständig zu schließen.

3. Heftmaschine nach Anspruch 1 oder 2, bei der mindestens einer von dem Amboß und dem Stößel wenigstens teilweise aus einem polymeren Material bestehen.

4. Heftmaschine nach einem der vorhergehenden Ansprüche, bei der der im allgemeinen U-förmige mittlere Abschnitt (12) der Klammer (10) im allgemeinen gerade Endteile besitzt, und die gebogenen Stirnflächen auf die im allgemeinen mit den Endteilen der Klammer (10) fluchtenden äußeren Abschnitte (16) der Klammer (10) Kräfte ausüben, die auf die Seiten derjenigen Endteile wirken, die der Mitte des mittleren Abschnitts (12) gegenüberliegen.

## Revendications

1. Agrafeuse, apte à fermer une agrafe (10) en fil métallique à l'état ouvert, qui est destinée à suturer un tissu vivant et est du type comprenant une partie centrale (12), sensiblement en U et comportant des extrémités opposées et au moins une partie courbe, et des parties extérieures (16) se terminant par des pointes aiguës (18), les parties (19) de chaque partie extérieure (16) situées à l'opposé de la pointe aigué (18 ) étant disposées approximativement à angle droit vis-à-vis d'un premier coin (22) et étant reliées à l'endroit de ce premier coin (22) à une extrémité de la partie centrale (12), l'agrafe étant agencée de façon à pouvoir être fermée par un pliage rendant sensiblement rectiligne la partie courbe de la partie centrale (12), au voisinage de chacune des parties extérieures (16), de sorte que ces parties extérieures (16) peuvent pénétrer dans le tissu vivant et le rassembler, fermeture au cours de laquelle les pointes aiguës (18) des parties extérieures (16) se déplacent à des positions adjacentes sensiblement suivant le même alignement l'une avec l'autre de façon à fournir une agrafe (10) fermée, l'agrafeuse comprenant:
une semelle (32) comportant des surfaces de support (34) espacées et un logement (36) situé entre ces surfaces de support (34) et apte à recevoir une zone centrale de la partie centrale (12) sensiblement en U de l'agrafe (10),
un poussoir (40) comprenant une partie d'extrémité, en une matière élastiquement flexible, qui comporte deux parties séparées (42) présentant chacune une surface d'extrémité apte à venir en prise sur l'agrafe (10) à l'état ouvert, les surfaces d'extrémité comportant des parties extérieures (46) espacées et des parties intérieures (48) disposées en retrait vis-à-vis des parties extérieures (46) et s'étendant d'une manière pratiquement transversale vis-à-vis du poussoir (40), les surfaces d'extrémité étant courbes, des parties extérieures (46) aux parties intérieures (48), de façon à former des parties de surface courbes (50) opposées et aptes à venir en prise le long des parties extérieures (16) de l'agrafe (10) ouverte, les jonctions entre les parties de surface courbes (50) et les parties de surface intérieures (48) formant des alvéoles aptes à venir en prise sur les premiers coins (22) de l'agrafe (10) lorsque sa partie centrale (12) est partiellement redressée et les parties de surface intérieures (48) étant disposées de façon à redresser la partie centrale (12) en travers de la semelle (32) et de façon à compléter la fermeture de l'agrafe (10), et
des moyens permettant de déplacer le poussoir (40) vers la semelle (32) de façon à fermer l'agrafe (10) autour de la semelle (32) avec la partie centrale (12) de l'agrafe (10) soutenue en appui sur les surfaces de support (34) espacées, en réalisant successivement les opérations consistant:
(1) à appliquer les parties de surface d'extrémité courbes (50) en appui sur les parties extérieures (16) de l'agrafe (10) de façon à faire s'écarter élastiquement les parties (42) du poussoir (40) et de façon à appliquer des forces à la fois suivant la direction longitudinale et suivant la direction transversale du poussoir (40) en vue de redresser partiellement la partie centrale (12) de l'agrafe (10),
(2) à appliquer ensuite les surfaces (50, 48) formant les alvéoles, en appui sur les premiers coins (22) de l'agrafe (10), de façon à redresser sensiblement la partie centrale (12) de cette agrafe (10) et
(3) à faire alors venir les parties de surface intérieures (48) du poussoir (40) en prise sur la partie centrale (12) de l'agrafe (10) de façon à redresser la partie centrale (12) et à compléter la fermeture de l'agrafe (10).

2. Agrafeuse comprenant :
une agrafe (10) en fil métallique à l'état ouvert, qui est destinée à suturer un tissu vivant, cette agrafe (10) à l'état ouvert étant du type comprenant une partie centrale (12), sensiblement en U et comportant des extrémités opposées et au moins une partie courbe, et des parties extérieures (16) se terminant par des pointes aiguës (18), des parties (19) de chaque partie extérieure (16) situées à l'opposé de la pointe aiguë (18 ) étant disposées approximativement à angle droit vis-à-vis d'un premier coin (22) et étant reliées à l'endroit de ce premier coin (22) à une extrémité de la partie centrale (12), l'agrafe étant agencée de façon à pouvoir être fermée par un pliage rendant sensiblement rectiligne la partie courbe de la parlie centrale (12), au voisinage de chacune des parties extérieures (16), de sorte que ces parties extérieures (16) peuvent pénétrer dans le tissu vivant et le rassembler, fermeture au cours de laquelle les pointes aiguës (18) des parties extérieures (16) se déplacent à des positions adjacentes sensiblement suivant le même alignement l'une avec l'autre de façon à fournir une agrafe (10) fermée,
une semelle (32) comportant des surfaces de support (34) espacées et un logement (36) situé entre ces surfaces de support (34) et apte à recevoir une zone centrale de la partie centrale (12) sensiblement en U de l'agrafe (10),
un poussoir (40) comprenant une partie d'extrémité, en une matière élastiquement flexible, qui comporte deux parties séparées (42) présentant chacune une surface d'extrémité apte à venir en prise sur l'agrafe (10) à l'état ouvert, les surfaces d'extrémité comportant des parties extérieures (46) espacées et des parties intérieures (48) disposées en retrait vis-à-vis des parties extérieures (46) et s'étendant d'une manière pratiquement transversale vis-à-vis du poussoir (40), les surfaces d'extrémité étant courbes, des parties extérieures (46) aux parties intérieures (48), de façon à former des parties de surface courbes (50) opposées et aptes à venir en prise le long des parties extérieures (16) de l'agrafe (10) ouverte, les jonctions entre les parties de surface courbes (50) et les parties de surface intérieures (48) formant des alvéoles aptes à venir en prise sur les premiers coins (22) de l'agrafe (10) lorsque la partie centrale (12) est partiellement redressée et les parties de surface intérieures (48) étant disposées de façon à redresser la partie centrale (12) en travers de la semelle (32) et de façon à compléter la fermeture de l'agrafe (10), et
des moyens permettant de déplacer le poussoir (40) vers la semelle (32) de façon à fermer l'agrafe (10) autour de la semelle (32) avec la partie centrale (12) de l'agrafe (10) soutenue en appui sur les surfaces de support (34) espacées, en réalisant successivement les opérations consistant:
(1) à appliquer les parties de surface d'extrémité courbes (50) en appui sur les parties extérieures (16) de façon à faire s'écarter élastiquement les parties (42) du poussoir (40) et de façon à appliquer des forces à la fois suivant la direction longitudinale et suivant la direction transversale du poussoir (40) en vue de redresser partiellement la partie centrale (12),
(2) à appliquer ensuite les surfaces (50, 48) formant les alvéoles, en appui sur les premiers coins (22) de l'agrafe (10), de façon à redresser sensiblement la partie centrale (12) et
(3) à faire alors venir les parties de surface intérieures (48) du poussoir (40) en prise sur la partie centrale (12) de façon à redresser la partie centrale (12) et à compléter la fermeture de l'agrafe (10).

3. Agrafeuse selon la revendication 1 ou 2, dans laquelle au moins l'un des deux éléments constitués par la semelle et le poussoir est au moins partiellement en une matière polymère.

4. Agrafeuse selon l'une quelconque des revendications précédentes, dans laquelle la partie centrale (12) sensiblement en U de l'agrafe (10) comporte des parties d'extrémité sensiblement rectilignes et les lesdites surfaces d'extrémité courbes appliquent, sur les parties extérieures (16) de l'agrafe (10), des forces qui sont sensiblement dans l'alignement des parties d'extrémité de l'agrafe (10) et agissent sur les côtés de ces parties d'extrémité qui sont situés à l'opposé de la zone centrale de la partie centrale (12).
